# EUROPEAN PATENT SPECIFICATION

(11) **EP 3 110 460 B1**
(45) Date of publication and mention of the grant of the patent: **26.12.2018**
(21) Application number: 15710884.6
(22) Date of filing: 30.01.2015
(51) Int. Cl.: A61L 2/24, A61B 90/70, A61L 2/26, A61B 1/00, A61L 2/18, G08B 13/22

(54) **DEVICE TO PROCESS AND TRANSPORT MEDICAL INSTRUMENTS IN A PROTECTED MANNER**
VORRICHTUNG FÜR BEHANDLUNG UND TRANSPORT MEDIZINISCHER INSTRUMENTEN IN EINER GESCHÜTZTEN WEISE
DISPOSITIF POUR TRAITER ET TRANSPORTER DES INSTRUMENTS MÉDICAUX DE FAÇON PROTÉGÉE

(30) Priority: 30.01.2014 IT UD20140013
(43) Date of publication of application: 04.01.2017
(73) Proprietor: Steelco S.p.A., 31039 Riese Pio X (IT)
(72) Inventor: ZARDINI, Fabio, 31033 Castelfranco Veneto (IT)
(74) Representative: Petraz, Davide Luigi
(86) International application number: PCT/IB2015/050709
(87) International publication number: WO 2015/114578

(56) References cited:
- EP-A1- 2 968 633
- WO-A1-2005/056060
- WO-A2-03/072147
- WO-A2-2013/060667

## Description

### FIELD OF THE INVENTION

Forms of embodiment of the present invention concern a device to process, in particular to wash, sterilize and dry medical instruments and to transport them in a protected manner inside a hospital or surgery, in particular flexible endoscopes, both during their washing and processing and afterward. In particular, the device according to the present invention can be used to wash and transport, in a protected manner, medical instruments of the tubular type made of various materials, in particular metal, plastic and/or rubber, used in medicine to explore and examine using optical apparatuses or to empty natural cavities of the organism, and introduced naturally or artificially, such as colonoscopes, bronchoscopes and gastroscopes.

### BACKGROUND OF THE INVENTION

Flexible endoscopes, used to examine internal parts of the human body or animals, are known. Endoscopic instruments typically include a flexible operating tube, also called insertion tube, normally with a diameter from 0.5 to 20 mm and a length from 300 to 4000 mm, suitable to be inserted into the patient's body, with a length and diameter that vary according to applications. Channels may be disposed or made inside the flexible operating tube, of various sizes and for various uses, for example for biopsies, TV camera inspections, for lighting, to irrorate air and/or water. Because of the purposes for which they are used, the endoscopes must be washed and disinfected carefully after use.

Washer machines are known, for washing medical instruments such as endoscopes used during clinical practice. Known machines of this type are described for example in the European patent EP-B-1.949.868 (EP'868) and in the European patent application EP-A-2.445.438 (EP'438).

Known washer machines typically include a washing chamber, in which the complete washing is carried out of both the external surfaces and internal surfaces and the channels of the endoscopic instrument, using washing liquids such as water and detergents or chemical disinfectants, for example based on glutaraldehyde or peracetic acid.

The external surface of the endoscopic instrument is typically treated by immersing it in a container, for example an auxiliary pipe, positioned in the washing chamber and connected to the washing circuit by a sleeve. The treatment can also be done using a washing device as described for example in EP'868 or EP'438.

The internal surfaces are treated by connecting flexible tubes to the internal channels of the endoscopic instrument.

A container is normally provided, to load the endoscopic instrument inside the washing chamber, in which the endoscopic instrument is positioned. An example of a loading container is described in EP'868 or EP'438.

It is a strongly felt need in this field to guarantee that the endoscopic instrument, or medical instrument in general, maintains its sterility even after treatment has taken place, and also when it is transported to its place of use, or during storage in suitable storehouses while waiting to be taken to its place of use.

EP-B-1.696.969 describes an apparatus for re-processing endoscopes equipped with several channels, held together by a sheath, which includes in its essential components a processing machine and a case that can be hermetically closed and that forms a chamber to receive an endoscope to be re-processed.

When the re-processing cycle of the endoscope has finished, the case is removed from the machine, with the endoscope inside, and taken by hand to its place of use.

The case, with the endoscope, can reach an overall weight of about 8 kilos or more. Therefore, manual transport to its place of use can be tiring, especially because, in medium to large hospital complexes, the machine can be several hundred meters from the place where the endoscope will be used.

The international application PCT/IB2013/061107 describes a machine for re-processing an endoscope that allows to facilitate the transport of the endoscope in the various sites where it is used and which guarantees the sterility of the endoscope contained in the chamber after re-processing also during transport to its place of use. The sterilization machine includes a fixed part and a mobile part which can be moved away from, nearer to, and reciprocally coupled with the fixed part and includes a tank suitable to receive the endoscope to be re-processed and a lid to hermetically close the tank.

These known solutions can satisfy the need to protect the endoscopic instrument once treated, for example during transport to its place of use.

However, none of the known solutions is able to prevent the risk of an endoscopic instrument, or medical instrument in general, once treated and sterilized and put in a suitable hermetically closed transport and protection device as described above, from being exposed, either voluntarily or inadvertently, to polluting environmental conditions, or in any case conditions that compromise the sterility obtained, subsequently and clearly restoring suitable transport or storage conditions.

Indeed it may be sufficient for the transport and protection device to be opened, inadvertently or not, and inevitably the medical instrument will no longer be sterile. Re-closing the transport and protection device, it may be possible to simulate that it was never opened after sterilization, in which case the final user may not be aware that the medical instrument is no longer sterile, but has been altered in some way, and will therefore use a medical instrument that in practice is not fit for purpose, and hence is potentially dangerous for the patient's health.

Documents WO-A-2013/060667 and WO-A-03/072147 are also known, which describe containers for objects to be sterilized, provided with a closing door or device and able to be put in an autoclave for steam treatment. These known containers are provided with an indicator to indicate that the closing door or device has been opened, or an indicator to indicate its sterility, coordinated with the opening of the closing door or device. However, these known containers are not very reliable and/or are difficult to use.

Document WO-A-2005056060 further discloses a known system for washing, sterilizing and preserving endoscopes.

Document EP-A-2.968.633 discloses a sterilization container capable of providing an indication regarding whether or not surgical instruments sterilized in the container were properly sterilized.

There is therefore a need to perfect a device to process and transport medical instruments in a protected manner that can overcome at least one of the disadvantages of the state of the art.

In particular, there is a need to obtain a device to process and transport medical instruments, particularly flexible endoscopes, in a protected manner, which is able to indicate to the final user whether, after re-processing and sterilization and during transport and possible storage, the medical instrument has maintained its sterility, having been correctly protected, or whether it has been exposed to non-sterile conditions and therefore is no longer suitable for medical use and must be sterilized again.

The Applicant has devised, tested and embodied the present invention to overcome the shortcomings of the state of the art and to obtain these and other purposes and advantages.

### SUMMARY OF THE INVENTION

The present invention is set forth and characterized in the independent claims, while the dependent claims describe other characteristics of the invention or variants to the main inventive idea.

In accordance with the above purposes, forms of embodiment described here concern a device, which overcomes the limits of the state of the art and eliminates the defects present therein, to process and transport medical instruments in a protected manner. In some forms of embodiment, the device includes a containing body, in which a medical instrument is placed, and a closing body.

The containing body and the closing body are configured mobile with respect to each other, in order to define a hermetically closed condition and an open condition.

In accordance with some forms of embodiment, the device includes at least one sensor configured to automatically detect a variation between the open condition and the hermetically closed condition and to transmit a corresponding signal, and an electronic state display configured to receive the signal from the sensor and to display toward the outside information regarding the current state associated specifically to the signal received.

Forms of embodiment described here also concern a device to process and transport medical endoscopic instruments in a protected manner, comprising a containing body, provided with a housing in which to place a medical endoscopic instrument, and a closing body, in which the containing body and the closing body are configured mobile with respect to each other, in order to define a hermetically closed condition and an open condition. According to one form of embodiment, the device includes:
- one or more hydraulic connectors suitable to work with liquids under pressure and connectable on one side to hydraulic exits of a washer machine for endoscopic instruments and on the other side: (i) to connectors of medical endoscopic instruments placed in said housing in order to reprocess the internal surfaces of said endoscopic medical instruments; and (ii) to the interior of said housing in order to reprocess the external surfaces of said medical endoscopic instruments;
- at least one sensor configured to automatically detect a variation between the open condition and the hermetically closed condition and to transmit a corresponding signal, said sensor being a position sensor and comprising at least two sensor elements, of which one sensor element disposed on the containing body and one sensor element disposed on the closing body; said at least two sensor elements are disposed in a coordinated and aligned position so that in the closed condition of the closing body they are in proximity, while in the open condition of the closing body they are distanced from each other, the reciprocal position of the two sensor elements varying in correlation to the passage from closed condition to open condition and vice versa, said variation being directly associated to the opening of said device, providing transmitting said corresponding signal;
- an electronic state display configured to receive said signal from the sensor and to display toward the outside information regarding the current state associated specifically to the signal received, said electronic state display being provided with a processing unit configured at least to perform a processing of the signal received from the sensor and to command a display of information regarding a corresponding state.
- a setting member configured to set a state of completed processing of the medical endoscopic instrument by transmitting a corresponding signal to the electronic state display.

According to other forms of embodiment, the electronic state display is configured to display at least a sterile state of the medical instrument associated with the hermetically closed condition and a non-sterile state of the medical instrument associated with the open condition and/or in the case of passage from the hermetically closed condition to the open condition.

According to other forms of embodiment, the electronic state display is provided on the closing body, or on the containing body.

According to other forms of embodiment, the device to process and transport medical instruments in a protected manner comprises a source of electric energy on board, configured to autonomously feed electric energy at least to the electronic state display.

According to other forms of embodiment, the electronic state display comprises one or more selectively activated light sources.

According to other forms of embodiment, the electronic state display comprises a display screen.

According to other forms of embodiment, the display screen is touch sensitive.

According to other forms of embodiment, the electronic state display is configured to display information for each state by means of a specific letter, combination of letters, writings and/or a graphical sign and/or a symbol or combination of symbols and/or one or more dedicated colors or other visual/optical signals and combinations thereof.

According to other forms of embodiment, the electronic state display comprises a user input interface.

According to other forms of embodiment, the electronic state display is configured to display information regarding the specific state in a manner that is non-reversible and/or non-modifiable without authorization.

According to other forms of embodiment, the sensor is chosen from either a contact sensor or a proximity sensor.

According to other forms of embodiment, the setting member comprises an external connector or socket, able to support the transmission and reception of an electric power signal, or data, or a combination of electric power and data signal.

According to other forms of embodiment, the setting member comprises the input interface.

Forms of embodiment described here also concern a method to process and transport medical endoscopic instruments in a protected manner. According to one form of embodiment the method includes:
- placing a medical endoscopic instrument in a housing of a containing body and closing the containing body by means of a closing body mobile with respect to the containing body in order to define a hermetically closed condition and an open condition.

The method also includes:
- supplying one or more processing fluids to the housing by means of one or more hydraulic connectors suitable to work with liquids under pressure and connectable on one side to hydraulic exits of a washer machine for endoscopic instruments and on the other side: (i) to connectors of medical endoscopic instruments placed in said housing in order to reprocess the internal surfaces of said endoscopic medical instruments; and (ii) to the interior of said housing in order to reprocess the external surfaces of said medical endoscopic instruments;
- automatically detecting a variation between the open condition and the hermetically closed condition and transmitting a corresponding signal by means of at least one sensor, the sensor being a position sensor and comprising at least two sensitive elements, of which one sensitive element disposed on the containing body and one sensitive element disposed on the closing body; said at least two sensor elements being disposed in a coordinated and aligned position so that in the closed condition of the closing body they are in proximity, while in the open condition of the closing body they are distanced from each other, the reciprocal position of the two sensor elements varying in correlation to the passage from closed condition to open condition and vice versa, said variation being directly associated to the opening of said device, providing transmitting said corresponding signal;
- receiving the signal from the sensor and displaying toward the outside information regarding the current state associated specifically to the signal received by means of an electronic state display, the electronic state display being provided with a processing unit that at least performs a processing of the signal received from the sensor and commands a display of information regarding a corresponding state;
- setting a state of completed processing of the medical endoscopic instrument by means of a setting member, transmitting a corresponding signal to the electronic state display.

These and other aspects, characteristics and advantages of the present disclosure will be better understood with reference to the following description, drawings and attached claims. The drawings, which are integrated and form part of the present description, show some forms of embodiment of the present invention, and together with the description, are intended to describe the principles of the disclosure.

The various aspects and characteristics described in the present description can be applied individually where possible. These individual aspects, for example aspects and characteristics described in the attached dependent claims, can be the object of divisional applications.

It is understood that any aspect or characteristic that is discovered, during the patenting process, to be already known, shall not be claimed and shall be the object of a disclaimer.

### BRIEF DESCRIPTION OF THE DRAWINGS

These and other characteristics of the present invention will become apparent from the following description of some forms of embodiment, given as a non-restrictive example with reference to the attached drawings wherein:
- fig. 1 is a perspective view of a device to process and transport medical instruments in a protected manner in a closed condition in accordance with forms of embodiment described here;
- fig. 2 is a perspective view of a device to process and transport medical instruments in a protected manner in an open condition in accordance with forms of embodiment described here;
- fig. 3 is a perspective view of a device to process and transport medical instruments in a protected manner in a closed condition in accordance with other forms of embodiment described here;
- fig. 4 is a perspective view of a device to process and transport medical instruments in a protected manner in an open condition in accordance with other forms of embodiment described here.

To facilitate comprehension, the same reference numbers have been used, where possible, to identify identical common elements in the drawings. It is understood that elements and characteristics of one form of embodiment can conveniently be incorporated into other forms of embodiment without further clarifications.

### DETAILED DESCRIPTION OF FORMS OF EMBODIMENT

We shall now refer in detail to the various forms of embodiment of the present invention, of which one or more examples are shown in the attached drawing.

Each example is supplied by way of illustration of the invention and shall not be understood as a limitation thereof. For example, the characteristics shown or described insomuch as they are part of one form of embodiment can be adopted on, or in association with, other forms of embodiment to produce another form of embodiment. It is understood that the present invention shall include all such modifications and variants.

Before describing these forms of embodiment, we would also clarify that the present description is not limited in its application to the details of the construction or disposition of the components as described in the following description using the attached drawings. The present description can provide other forms of embodiment and achieved or executed in other various ways. We also clarify that the phraseology and terminology used here are used for the purposes of description and must not be considered as restrictive. The use of words such as "including", "comprising", "having" and variations thereof is intended to include the elements listed after them and their equivalents, and also additional elements. Unless otherwise specified, the words "mounted", "connected", "supported" and "coupled" and their variations are used in a wide sense and include both direct and indirect assemblies, connections, supports and couplings. Furthermore, the words "connected" and "coupled" are not limited to physical or mechanical connections or couplings.

The forms of embodiment described here refer to a device to process and transport medical instruments in a protected manner.

It is understood that a medical instrument as used in forms of embodiment described here can be a medical instrument usable in a hospital or surgery, in particular a flexible endoscope. In general, medical instruments usable in forms of embodiment described here can be medical instruments of the tubular type made of various materials, in particular metal, plastic and/or rubber, used in medicine to explore or examine using optical apparatuses, or to empty natural cavities in the body and introduced naturally or artificially, such as for example colonoscopes, bronchoscopes and gastroscopes.

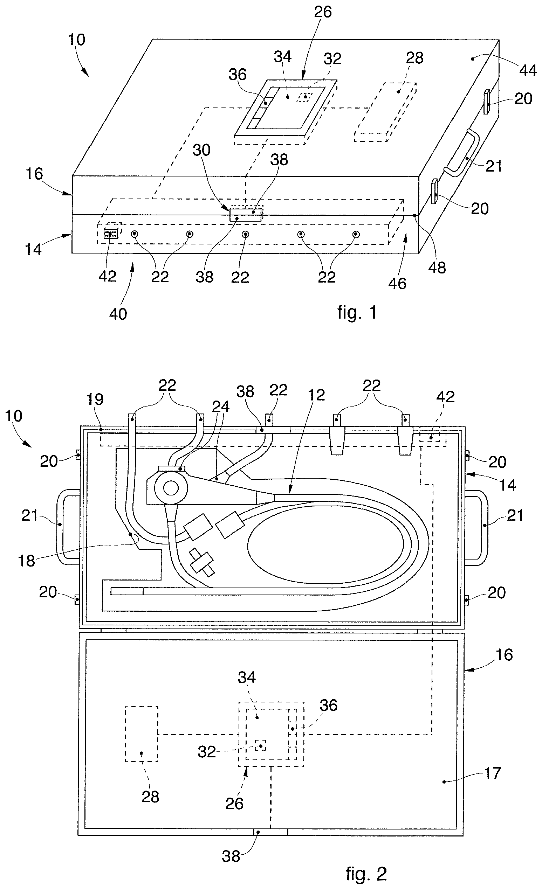

Forms of embodiment described here can provide a device 10 to process and transport medical instruments 12 in a protected manner (see fig. 2 for example), such as endoscopic instruments or endoscopes, in accordance with the present description.

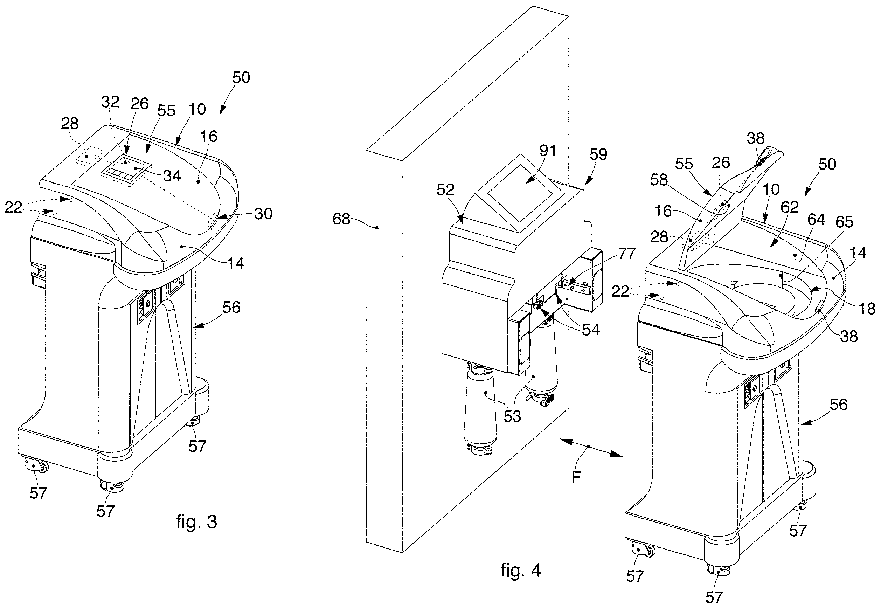

The device 10 can include a containing body 14 and a closing body 16. The containing body 14 can provide a housing 18 inside it (see figs. 2 and 4 for example) in which to place a medical instrument 12 to be processed.

The containing body 14 and the closing body 16 can be configured mobile with respect to each other, in order to define a closed condition of the device 10 and an open condition of the device 10.

In possible implementations, the containing body 14 and the closing body 16 can be mobile thanks to reciprocal hinging around an axis of rotation X. The hinging can be at the front or side, so that the opening/closing movement can for example be drop-down or like a book.

In the closed condition, the device 10 encloses a processing chamber inside it in which the re-processing and/or sterilization of the medical instrument 12 can take place, both of its external surfaces and its internal surfaces. The processing chamber can thus be defined by the housing 18, that is, by the bottom and the lateral walls that delimit it, and by the closing body 16, for example by one of its internal walls 17 (see fig. 2 for example). Typically, the processing, re-processing and/or sterilization operations of the medical instrument 12 can, and normally must, take place with the device 10 in the closed condition.

Typically, the device 10 is configured to guarantee the airtight seal in the closed condition. In possible implementations, perimeter packings 19 can be provided (see fig. 2 for example) to guarantee the hermetic seal of containing body 14 and closing body 16, in the closed condition. Moreover, clamping members 20 can be provided to clamp containing body 14 and closing body 16 from the outside. In this way, after the medical instrument 12 has been processed and sterilized, and thanks to the airtight seal of the closing, it keeps the desired conditions of sterility suitable for its subsequent uses.

The device 10 includes one or more entrances 22 for processing fluids, in particular washing, sterilization and drying, of the medical instrument 12 provided in the housing 18. The entrances 22 are typically connectable on one side to fluidic/hydraulic exits of a washer machine for endoscopic instruments, and on the other side, directly or indirectly, to the inside of the containing body 14. In particular, inside the containing body 14 the entrances 22 can be connected to connectors 24 of the medical instrument 12 placed therein, in order to reprocess the internal surfaces, just as they can open directly to the inside of the processing chamber, for example to process the external surfaces of the medical instrument 12.

The one or more entrances 22 are hydraulic connectors suitable to operate with liquids under pressure. For example, connectors of the rapid type can be provided. When the entrances 22 are not connected to the fluidic/hydraulic exits of a washer machine for endoscopic instruments and/or to the connectors 24 of the medical instrument 12, they are closed, preventing the passage of fluids or other inside the containing body 14, thus preserving the sterility thereof.

The entrances 22 can be provided on the containing body 14 and/or on the closing body 16, at the front or at the back, or again laterally, above or below, according to needs.

In accordance with the present description, the device 10 is provided externally with an electronic state display 26. The electronic state display 26 is configured to display a multiplicity of information correlated to a plurality of states associated to the device 10 or to the medical instrument 12 contained therein.

By virtue of its conformation, structure and/or position, the electronic state display 26 is directly, and without difficulty, visible from the outside for a final user who can thus immediately recognize the information that it transmits and can proceed accordingly.

In possible implementations, the electronic state display 26 can be configured to display at least a sterile state of the medical instrument 12, typically associated to the hermetically closed condition, and a non-sterile state of the medical instrument 12, associated to the open condition.

According to forms of embodiment described here, which can be combined with all the forms of embodiment described here, the electronic state display 26 can be configured to automatically change information displayed relating to a displayed state depending on the closed or open condition that the device 10 can assume on each occasion.

In this way, even if the device 10 is opened after the medical instrument 12 has been processed, re-processed and sterilized, and subsequently re-closed, altering the sterile condition of the medical instrument 12 itself, the final user can be directly informed of this, thanks to the appropriate state displayed automatically by the electronic state display 26.

In possible implementations, the electronic state display 26 can be provided on the closing body 16, for example in a front, upper or lateral position.

In other possible implementations, the electronic state display 26 can be provided on the containing body 14, in this case too for example in a front, upper or lateral position.

In accordance with possible forms of embodiment, the closing body 16 can be a closing lid.

In accordance with some forms of embodiment, which can be combined with all the forms of embodiment described here, the device 10 can include a source of electric energy 28 on board, configured to autonomously feed electric energy at least to the electronic state display 26. In this way it is possible to render the electronic state display 26 energy autonomous.

In accordance with some forms of embodiment, which can be combined with all the forms of embodiment described here, the source of electric energy 28 can be integrated or incorporated in the closing body 16.

Or, in other forms of embodiment, the source of electric energy 28 can be integrated or incorporated in the containing body 14.

For example, the source of electric energy 28 can be protected in a suitable housing chamber.

In possible implementations, the source of electric energy 28 can include a battery or primary or secondary battery, or electric load accumulator. Several batteries or primary or secondary batteries can be provided, in series, or a pack of batteries. Therefore the source of electric energy 28 may not be rechargeable, and in this case it is disposed of and replaced, or rechargeable, such as a buffer battery.

The device 10 is provided with a sensor 30 (see fig. 1 for example) configured to automatically detect a variation between the open condition and the closed condition of the device 10 and to transmit a corresponding signal to the electronic state display 26.

In accordance with some forms of embodiment, which can be combined with all the forms of embodiment described here, the electronic state display 26 can be configured to receive the signal from the sensor 30 and to process it, so as to display toward the outside, among possible displayable states, information on the current state of the device 10 and/or the medical instrument 12 contained therein, which current state is specifically associated to the signal received, so that the information displayed is received intuitively by the final user.

In possible implementations, the electronic state display 26 can be connected to the sensor 30 via cable, or by wireless connections.

In accordance with some forms of embodiment, which can be combined with all the forms of embodiment described here, the electronic state display 26 can be provided with a processing unit 32 configured at least to carry out the processing of the signal received and to command, accordingly, the display of a specific piece of information correlated to the corresponding state.

For example, the processing unit 32 can include a system controller, a microcontroller, a PLC (Programmable Logic Controller), a processor or microprocessor (CPU), provided with an EPROM (Erasable Programmable Read-Only Memory) card, or EEPROM (Electrically Erasable Programmable Read-Only Memory) card, or other similar electronic control/processing/memorization systems.

For example the system controller can include a central processing unit (CPU), a memory, and auxiliary circuits (or I/O). For example, the CPU can be any form of processor for computers that can be used in industrial regulations to control different chamber processes and hardware devices (such as conveyors, optical inspection units, motors, fluid delivery devices etc.) and to monitor the system and the chamber processes (such as substrate position, process times, detection signals etc.). The memory can be connected to the CPU and can be one or more of those readily available, such as RAM (Random Access Memory), ROM (Read Only Memory), floppy disc, hard disc, mass memory, or any other form of digital, local or remote storage. Possible software instructions and data can be encoded and memorized in the memory for example to command the CPU. The auxiliary circuits can also be connected to the CPU to help the processor in a conventional manner. The auxiliary circuits can include for example, at least one of: cache circuits, feed circuits, clock circuits, input/output circuitry, subsystems and suchlike.

In accordance with some forms of embodiment, which can be combined with all the forms of embodiment described here, the electronic state display 26 can include one or more light sources that can be selectively activated, for example a LED (Light Emitting Diode), or an array of LEDs.

In accordance with other forms of embodiment, which can be combined with all the forms of embodiment described here, the electronic state display 26 can include a display screen 34, for example a LED screen and/or a LCD (Liquid Crystal Display) screen.

In accordance with some forms of embodiment, which can be combined with all the forms of embodiment described here, the electronic state display 26 can be configured to display information for each state of the device 10 by means of a specific letter, combination of letters, writings and/or a graphical sign and/or a symbol or combination of symbols and/or one or more dedicated colors or other visual/optical signals and combinations thereof. In possible complementary variants the electronic state display 26 can possibly also be configured to supply a specific acoustic signal if the state of the device 10 is varied.

In accordance with some forms of embodiment, which can be combined with all the forms of embodiment described here, the electronic state display 26 can include a user input interface 36, provided with buttons or similar input members for example, so that the user can interact with the electronic state display 26 to modify and set the state displayed manually. For example, the display screen 34 can be the touch-sensitive type and thus make up the input interface 36 itself. In the interests of security, it can be provided that the input interface 36 is only accessible to authorized users, using passwords or other authentication and recognition systems.

In accordance with some forms of embodiment, which can be combined with all the forms of embodiment described here, the electronic state display 26 can be configured to display information on the specific state of the device 10 in a manner that is non-reversible and non-modifiable without authorization, in particular by users who are not authorized. This is to prevent a user, if the device 10 is altered and therefore the electronic state display 26 indicates that the device 10 has been opened and therefore indicates a lack of sterility of the medical instrument 12, from improperly restoring the display of another state on the electronic state display 26.

In accordance with some forms of embodiment, which can be combined with all the forms of embodiment described here, the sensor 30 can be selected from a mechanical, electromechanical, electric, electronic, magnetic, optical sensor or a combination of these.

The sensor 30 includes at least two sensor elements 38, for example associated, respectively, one to the containing body 14 and one to the closing body 16.One sensor element 38 is disposed on the containing body 14 and one sensor element 38 is disposed on the closing body 16. The sensor elements 38 are disposed in a coordinated and aligned position so that in the closed condition of the closing body 16 they are in proximity, advantageously adjacent, while in the open condition of the closing body 16 they are distanced from each other. In this way, in the passage between the closed condition and the open condition, and vice versa, the reciprocal position of the two sensor elements 38 varies in a reliably detectable and repeatable way, and this variation, being correlated to the passage from closed condition to open condition and vice versa, can be directly associated to the opening of the device 10, transmitting a corresponding signal to the electronic state display 26.

The sensor 30 is a position sensor, for example a contact sensor or a proximity sensor or similar sensor member. The position sensor can operate as a transducer, being configured to detect the presence or position of a physical object in the immediate vicinity or in contact with respect to a sensitive side of the sensor and to transmit a correlated signal.

In the case of a contact sensor, this can make use of a tactile property, of force or collision. The contact sensor can be electric, piezoelectric, inductive, capacitive, magnetic, ultrasound or optical.

In the case of a proximity sensor, it can be for example inductive, capacitive, magnetic, ultrasound or optical.

In accordance with some forms of embodiment, which can be combined with all the forms of embodiment described here, the device 10 includes a setting member 40 configured to set the state of processing, re-processing and/or sterilization that has taken place on the medical instrument 12 with the device 10 closed, transmitting a corresponding signal to the electronic state display 26.

The setting member 40 can thus be configured to intervene on the electronic state display 26, so that at the end of the processing, re-processing and/or sterilization operations, a state of sterility is set and displayed on the electronic state display 26.

In possible implementations, the setting member 40 can be configured to proceed automatically to set the state of processing that has taken place.

For example, the setting member 40 can be formed by a connector or socket 42, able to support the reception and transmission of an electric feed signal or a data signal, or a combination of electric feed and data signal. The connector or socket 42 can be provided externally to the device 10, for example, in correspondence to the entrances 22. The connector or socket 42 can be connected to the electronic state display 26 to supply the signal. In this way, the electric connector or socket 42 can be engaged with a mating socket or connector (not shown) provided on the washer machine with which the device 10 is used, to coincide with the connection of the entrances 22 and, at the end of processing, when the entrances 22 are disconnected, the connector or socket 42 can also be disengaged, or end-of-cycle information can be received. The variation between engagement and disengagement of the connector or socket 42, or the end-of-cycle information, can be used to supply an electric and/or data signal to the electronic state display 26 so that it automatically sets the display of process already done.

In other possible implementations, the automatic setting member 40 can be configured to be manually activated by an authorized user who, at the end of the processing, re-processing operations can intervene to set the completed processing state. For example, the automatic setting member 40 can include the input interface 36, possibly also in the version in which it is formed by the display screen 34 in the touch-sensitive version.

In possible implementations, once the device 10, after the sterilization cycle, is opened, the automatic setting member 40 can no longer be re-activated or reset to set a "sterile" state of the device 10 and the medical instrument 12 contained therein, unless the device 10 is subjected to another sterilization cycle that restores the sterile condition, in which case the procedure described above is followed. This is to prevent, in the event of an undesired opening and loss of sterility, the possibility of by-passing, in an unnoticed, fraudulent or undesired way, the control and the indication of sterility of the device 10. In this way the device 10 cannot be set to "sterile" by mistake using the automatic setting member 40, despite the electronic state display 26 indicating the loss of "sterility" due to the opening of the device 10.

Figs. 1 and 2 are used to describe a plurality of forms of embodiment, which can be combined with all the forms of embodiment described here, in which the device 10 is essentially configured as a portable case and in which the containing body 14 can be a box-like body, generally of a parallelepiped shape, while the closing body 16 can be a closing lid of the case, for example this too of a parallelepiped shape, hinged to the box-like body. In this case, gripping handles 21 can be provided on one or both sides of the case, typically to facilitate maneuvering and transport.

For example, in some forms of embodiment, the electronic state display 26 can be provided on an upper face 44 of the closing body 16 (see fig. 1).

In some forms of embodiment, the on-board electric energy source 28 according to the present description can be provided integrated or incorporated with the closing lid of the case that makes up the closing body 16.

Moreover, the sensor 30 can be provided for example on a lateral face 46 of the device 10, essentially astride a join profile 48 between the containing body 14 and the closing body 16. In particular, for example, it can be provided that the sensor 30 has the sensor elements 38 on one side and the other with respect to the join profile 48, in an aligned position.

The setting member 40 can be a connector or socket 42 located on a lateral face 46 of the device 10, for example on the containing body 14 (see fig. 1), or on the closing body 16.

Figs. 3 and 4 are used to describe a plurality of forms of embodiment that can be combined with all the forms of embodiment described here, in which the device 10 is configured essentially as a mobile part 50 of a sterilizing machine including at least a fixed part 59 and a mobile part 50 containing at least a medical instrument 12 (not shown in figs. 3 and 4, however comparable for example to that described using figs. 1 and 2).

The fixed part 59 includes a first fixed frame 52 on which at least part of a fluid-dynamic circuit to feed and/or discharge fluids is mounted. In accordance with possible forms of embodiment, injection means 53 can be mounted on the fixed part for the controlled injection of fluids into the fluid-dynamic circuit. Moreover, the fixed part 59 can be installed on a support wall 68 or on another similar or comparable support wall suitable to the purpose.

The mobile part 50 is movable reciprocally nearer to/away from the fixed part 59, as indicated by the arrow F in fig. 4, and is selectively able to be coupled to the latter.

The mobile part 50 also includes a tank 62 that acts as or defines the containing body 14. The tank 62 is provided with a cavity or housing 65 that acts as or defines the housing 18, suitable to receive the medical instrument 12 to be re-processed.

The mobile part 50 also includes at least one lid 55, that acts as or defines the closing body 16, suitable to hermetically close the tank 62.

In particular, the tank 62 is provided with at least one aperture 64 through which to insert and remove the medical instrument 12.

The tank 62 defines, when the lid 55 is closed, the processing chamber in which the medical instrument 12 is washed, dried, and/or sterilized.

Therefore, with respect to the forms of embodiment in figs. 1 and 2, in the forms of embodiment described using figs. 3 and 4, it can be provided that the closing body 16 is defined or made by the lid 55, that the containing body 14 is defined or made by the tank 62 and that the housing 18 is defined or made by the cavity 65 of the tank 62.

The lid 55 is suitable to assume at least a first position, or closed condition, shown for example in fig. 3, in which an internal wall 58 of the lid 55 and the cavity 65 of the tank 62 form said processing chamber, and a second position, or open condition, shown for example in fig. 4, in which the lid 55 is open and the tank 62 is accessible so that the medical instrument 12 can be replaced or removed.

The closing body 16, and therefore the lid 55, can be hinged to the containing body 14 that defines the tank 62 so that it can be rotated to move from the first to the second position, or vice versa.

The mobile part 50 includes a fluid-dynamic circuit configured to be connected, during use, to the medical instrument 12 and to the tank 62. The fluid-dynamic circuit can include connection means, for example, sockets or entrances 22, to which to connect the connectors of the medical instrument 12 (see fig. 4).

The mobile part 50 includes a first mobile frame 56 configured to support at least the containing body 14 with the tank 62, the lid 55 and the fluid-dynamic circuit.

The frame 56 of the mobile part supplies in itself a support for the containing body 14 with the tank 62 and the lid 55, which makes it unnecessary to have a support plane in the place in which the medical instrument 12 is used. A similar argument is also valid for when it is necessary to dispose the medical instrument 12 in the tank 62 before it is re-processed.

In possible solutions of the invention, it can be provided that the frame 56 of the mobile part is made in a single body at least with the containing body 14 and the corresponding tank 62. This allows to obtain an extremely solid and resistant structure for its movement.

In other solutions it can be provided that the containing body 14 and the corresponding tank 62 are separate elements from the frame 56 of the mobile part and subsequently mounted integrated on the latter.

The frame 56 of the mobile part can develop in a vertical direction to facilitate access to the tank 62 by an operator, to promote ergonomic use, and can also be telescopic, to vary at least the positioning height of the tank 62.

In accordance with some forms of embodiment, the frame 56 of the mobile part can be provided with rolling means 57 for the movement of the mobile part 50, as indicated by the arrow F. In particular, the rolling means 57 support the frame 56 of the mobile part and allow it to slide on a support plane.

The rolling means 57 allow to move the mobile part 50 nearer to or farther away from the fixed part 59. In this way the mobile part 50 can be transferred from the fixed part 59 to the room where the endoscopic instrument will be used, and vice versa, without having to make any lifting effort.

The rolling means 57 can include wheels, pirouetting wheels, rollers, castors, balls, bearings or similar components suitable to the purpose.

In possible implementations, the fluid-dynamic circuit of the mobile part can be provided with a plurality of entrances 22 mounted on the frame 56 of the mobile part and selectively able to be coupled removably with connectors 54 mounted on the frame 52 of the fixed part 59 to obtain the fluidic communication with the corresponding fluid-dynamic circuit of the fixed part 59 (see fig. 4).

In accordance with a possible form of embodiment, the coupled condition of the mobile part 50 with the fixed part 59 also determines a corresponding action of connecting the connectors 54 of the fixed part 59 and the entrances 22 of the mobile part 50.

In this way when the mobile part 50 is coupled to the fixed part 59, the two fluid-dynamic circuits can be connected to each other, allowing the process fluids to pass to and from the medical instrument 12 and to and from the tank 62. In particular, the two fluid-dynamic circuits are configured to allow at least one of either washing, sterilization liquids, purge gas, or gas to check the airtight seal to pass through them. Likewise, the two fluid-dynamic circuits can be configured to also allow the discharge of washing liquids present in the tank 62. When the entrances 22 are not connected to the connectors 54 and/or the connectors of the medical instrument 12, these are closed, preventing the passage of fluids or other inside the containing body 14, thus preserving its sterility.

Moreover, according to possible forms of embodiment of the present invention, the mobile part 50 and the fixed part 59 include anchoring means 77 configured to removably constrain the frame 52 of the fixed part 59 to the frame 56 of the mobile part 50, when the fixed part 59 and the mobile part 50 are coupled to each other.

The fixed part 59 can include a control and management device 91 (fig. 4), possibly governed by a user interface, and configured to control and manage the washing process of the medical instrument 12. The control and management device 91 can supply a start-of-cycle signal, and an end-of-cycle signal, the latter usable in the device 10 to set, manually or automatically, the display of completed treatment on the electronic state display 26.

In accordance with forms of embodiment described using figs. 3 and 4, the electronic state display 26 according to the present description can be provided externally on the lid 55 (see fig. 3 for example).

Alternatively, the electronic state display 26 can be provided on the tank 62 in an easily visible position for the user.

If the source of electric energy 28 is on board, according to the present description, it can be provided integrated into the lid 55, or in another zone of the tank 62 or the frame 56 of the mobile part.

Moreover, the sensor 30 according to the present description can be associated with the lid 55 and the tank 62, providing a sensor element 38 for example on the lid 55 and a sensor element 38 on the tank 62 (see fig. 4 for example).

Furthermore, the setting member 40 can be a connector or socket 42 provided at the back on the mobile part 50, for example in proximity to the entrances 22. The connector or socket 42, connecting for example to a mating socket or connector of the fixed part 59, can thus receive a useful signal from the fixed part 59, for example from the control and management device 91, or, as described above, the setting member 40 can be part of the input interface 36.

## Claims

1. Device to process and transport medical endoscopic instruments in a protected manner, comprising a containing body (14, 62), provided with a housing (18, 65) in which to place a medical endoscopic instrument (12), and a closing body (16, 55), in which the containing body (14, 62) and the closing body (16, 55) are configured mobile with respect to each other, in order to define a hermetically closed condition and an open condition, **characterized in that** it comprises:
- one or more hydraulic connectors (22) suitable to work with liquids under pressure and connectable on one side to hydraulic exits of a washer machine for endoscopic instruments and on the other side: (i) to connectors (24) of medical endoscopic instruments placed in said housing in order to reprocess the internal surfaces of said endoscopic medical instruments; and (ii) to the interior of said housing in order to reprocess the external surfaces of said medical endoscopic instruments;
- at least one position sensor (30) configured to automatically detect a variation between the open condition and the hermetically closed condition and to transmit a corresponding signal, said position sensor (30) comprising at least two sensor elements (38), of which one sensor element (38) disposed on the containing body (14) and one sensor element (38) disposed on the closing body (16), said at least two sensor elements (38) being disposed in a coordinated and aligned position so that in the closed condition of the closing body (16) they are in proximity, while in the open condition of the closing body (16) they are distanced from each other, the reciprocal position of the two sensor elements (38) varying in correlation to the passage from closed condition to open condition and vice versa, said variation being directly associated to the opening of said device, providing transmitting said corresponding signal;
- an electronic state display (26) configured to receive said signal from the sensor (30) and to display toward the outside information regarding the current state associated specifically to the signal received, said electronic state display (26) being provided with a processing unit (32) configured at least to perform a processing of the signal received from the sensor (30) and to command a display of information regarding a corresponding state;
- a setting member (40) configured to set a state of completed processing of the medical endoscopic instrument (12) by transmitting a corresponding signal to the electronic state display (26).

2. Device as in claim 1, **characterized in that** said electronic state display (26) is configured to display at least a sterile state of the medical endoscopic instrument (12) associated with the hermetically closed condition and a non-sterile state of the medical endoscopic instrument (12) associated with the open condition and/or in the case of passage from the hermetically closed condition to the open condition.

3. Device as in claim 1 or 2, **characterized in that** said electronic state display (26) is provided on the closing body (16), or on the containing body (14).

4. Device as in any claim hereinbefore, **characterized in that** it comprises a source of electric energy (28) on board, configured to autonomously feed electric energy at least to the electronic state display (26).

5. Device as in any claim hereinbefore, **characterized in that** the electronic state display (26) includes one or more selectively activated light sources.

6. Device as in any claim hereinbefore, **characterized in that** the electronic state display (26) comprises a display screen (34).

7. Device as in any claim hereinbefore, **characterized in that** said display screen (34) is touch sensitive.

8. Device as in any claim hereinbefore, **characterized in that** the electronic state display (26) is configured to display information for each state by means of a specific letter, combination of letters, writings and/or a graphical sign and/or a symbol or combination of symbols and/or one or more dedicated colors or other visual/optical signals and combinations of these.

9. Device as in any claim hereinbefore, **characterized in that** the electronic state display (26) comprises a user input interface (36).

10. Device as in claim 9, **characterized in that** said setting member (40) comprises said input interface (36).

11. Device as in any claim hereinbefore, **characterized in that** the electronic state display (26) is configured to display information regarding the specific state in a manner that is non-reversible and/or non-modifiable without authorization.

12. Device as in any claim hereinbefore, **characterized in that** the sensor (30) is chosen from either a contact sensor or a proximity sensor.

13. Device as in any claim hereinbefore, **characterized in that** said setting member (40) comprises an external connector or socket (42), able to support the transmission and reception of an electric power signal, or data, or a combination of electric power and data signal.

14. Device as in claim 13, **characterized in that** said connector or socket (42) can be connected to the electronic state display (26) to supply the signal, wherein the connector or socket (42) can be engaged with a mating socket or connector provided on a washer machine, to coincide with the connection of the connectors (22) and, at the end of processing, when the connectors (22) are disconnected, the connector or socket (42) can be disengaged, or end-of-cycle information can be received, further wherein the variation between engagement and disengagement of the connector or socket (42), or the end-of-cycle information, can be used to supply an electric and/or data signal to the electronic state display (26) so that it automatically sets the display of process already done.

15. Device as in any claim hereinbefore, **characterized in that** said connectors (22) are rapid type connectors.

16. Device as in any claim hereinbefore, **characterized in that** when said connectors (22) are not connected to hydraulic exits of a washer machine for endoscopic instruments and/or to connectors (24) of the medical endoscopic instrument (12), said connectors (22) are closed, preventing the passage of fluids or other inside the containing body (14).

17. Device as in any claim hereinbefore, **characterized in that** said device comprises perimeter packings (19) to guarantee the hermetic seal of containing body (14) and closing body (16), in the closed condition.

18. Method to process and transport medical endoscopic instruments in a protected manner, comprising:
- placing a medical endoscopic instrument (12) in a housing (18, 65) of a containing body (14, 62) and closing the containing body (14, 62) by means of a closing body (16, 55) mobile with respect to the containing body (14, 62) in order to define a hermetically closed condition and an open condition,
**characterized in that** the method comprises:
- supplying one or more processing fluids to the housing (18, 65) by means of one or more hydraulic connectors (22) suitable to work with liquids under pressure and connectable on one side to hydraulic exits of a washer machine for endoscopic instruments and on the other side: (i) to connectors (24) of medical endoscopic instruments placed in said housing in order to reprocess the internal surfaces of said endoscopic medical instruments; and (ii) to the interior of said housing in order to reprocess the external surfaces of said medical endoscopic instruments;
- automatically detecting a variation between the open condition and the hermetically closed condition and transmitting a corresponding signal by means of at least one sensor (30), said sensor (30) being a position sensor and comprising at least two sensor elements (38), of which one sensor element (38) disposed on the containing body (14) and one sensor element (38) disposed on the closing body (16), said at least two sensor elements (38) being disposed in a coordinated and aligned position so that in the closed condition of the closing body (16) they are in proximity, while in the open condition of the closing body (16) they are distanced from each other, the reciprocal position of the two sensor elements (38) varying in correlation to the passage from closed condition to open condition and vice versa, said variation being directly associated to the opening of said device, providing transmitting said corresponding signal;
- receiving said signal from the sensor (30) and displaying toward the outside information regarding the current state associated specifically to the signal received by means of an electronic state display (26), said electronic state display (26) being provided with a processing unit (32) that at least performs a processing of the signal received from the sensor (30) and commands a display of information regarding a corresponding state;
- setting a state of completed processing of the medical endoscopic instrument (12) by means of a setting member (40), transmitting a corresponding signal to the electronic state display (26).

## Patentansprüche

1. Vorrichtung zum Behandeln und Transportieren von medizinischen endoskopischen Instrumenten in einer geschützten Weise, aufweisend einen Aufnahmekörper (14, 62), der mit einem Gehäuse (18, 65) bereitgestellt ist, in welchem ein medizinisches endoskopisches Instrument (12) platzierbar ist, und einen Schließkörper (16, 55), wobei der Aufnahmekörper (14, 62) und der Schließkörper (16, 55) relativ zueinander bewegbar konfiguriert sind, um einen hermetisch geschlossenen Zustand und einen offenen Zustand zu definieren, **dadurch gekennzeichnet, dass** sie aufweist:
- einen oder mehrere hydraulische Verbinder (22), die geeignet sind, um mit unter Druck stehenden Flüssigkeiten zu arbeiten, und die verbindbar sind auf einer Seite mit hydraulischen Ausgängen einer Waschmaschine für endoskopische Instrumente und auf der anderen Seite: (i) mit Verbindern (24) von medizinischen endoskopischen Instrumenten, die in dem Gehäuse angeordnet sind, um die Innenflächen der endoskopischen medizinischen Instrumente wiederaufzubereiten, und (ii) mit dem Inneren des Gehäuses, um die Außenflächen der medizinischen endoskopischen Instrumente wiederaufzubereiten,
- wenigstens einen Positionssensor (30), der konfiguriert ist, um eine Variation zwischen dem offenen Zustand und dem hermetisch geschlossenen Zustand automatisch zu erfassen und ein korrespondierendes Signal zu senden, wobei der Positionssensor (30) aufweist wenigstens zwei Sensorelemente (38), von denen ein Sensorelement (38) an dem Aufnahmekörper (14) angeordnet ist und ein Sensorelement (38) an dem Schließkörper (16) angeordnet ist, wobei die wenigstens zwei Sensorelemente (38) in einer abgestimmten und ausgerichteten Position angeordnet sind, sodass sie im geschlossenen Zustand des Schließkörpers (16) benachbart sind, wohingegen sie im offenen Zustand des Schließkörpers (16) im Abstand voneinander sind, wobei die gegenseitige Position der beiden Sensorelemente (38) variiert in Korrelation zu dem Übergang von dem geschlossenen Zustand zu dem offenen Zustand und umgekehrt, wobei diese Variation direkt mit dem Öffnen der Vorrichtung assoziiert ist, bereitstellend das Senden des korrespondierenden Signals,
- eine elektronische Zustandsanzeige (26), die konfiguriert ist, um das Signal von dem Sensor (30) zu empfangen und zur Außenseite hin Informationen bezüglich des momentanen Zustands anzuzeigen, der speziell mit dem empfangen Signal assoziiert ist, wobei die elektronische Zustandsanzeige (26) bereitgestellt ist mit einer Verarbeitungseinheit (32), die konfiguriert ist, um wenigstens ein Verarbeiten des Signals, das von dem Sensor (30) empfangen wurde, durchzuführen und eine Anzeige von Informationen bezüglich eines korrespondierenden Zustands zu befehlen,
- ein Setzelement (40), das konfiguriert ist, um einen Zustand des komplettierten Behandelns des medizinischen endoskopischen Instruments (12) zu setzen durch Senden eines korrespondierenden Signals an die elektronische Zustandsanzeige (26).

2. Vorrichtung gemäß Anspruch 1, **dadurch gekennzeichnet, dass** die elektronische Zustandsanzeige (26) konfiguriert ist, um wenigstens einen Steril-Zustand des medizinischen endoskopischen Instruments (12), der mit dem hermetisch geschlossenen Zustand assoziiert ist, und einen Nicht-Steril-Zustand des medizinischen endoskopischen Instruments (12) anzuzeigen, der mit dem offenen Zustand assoziiert ist und/oder im Falle des Übergangs von dem hermetisch geschlossenen Zustand zu dem offenen Zustand.

3. Vorrichtung gemäß Anspruch 1 oder 2, **dadurch gekennzeichnet, dass** die elektronische Zustandsanzeige (26) an dem Schließkörper (16) oder an dem Aufnahmebehälter (14) bereitgestellt ist.

4. Vorrichtung gemäß irgendeinem vorigen Anspruch, **dadurch gekennzeichnet**, sie aufweist eine Quelle elektrischer Energie (28) an Bord, die konfiguriert ist, um elektrische Energie autonom zuzuführen an wenigstens die elektronische Zustandsanzeige (26).

5. Vorrichtung gemäß irgendeinem vorigen Anspruch, **dadurch gekennzeichnet, dass** die elektronische Zustandsanzeige (26) aufweist eine oder mehrere selektiv aktivierte Lichtquellen.

6. Vorrichtung gemäß irgendeinem vorigen Anspruch, **dadurch gekennzeichnet, dass** die elektronische Zustandsanzeige (26) aufweist einen Anzeigeschirm (34).

7. Vorrichtung gemäß irgendeinem vorigen Anspruch, **dadurch gekennzeichnet, dass** der Anzeigeschirm (34) berührungssensitiv ist.

8. Vorrichtung gemäß irgendeinem vorigen Anspruch, **dadurch gekennzeichnet, dass** die elektronische Zustandsanzeige (26) konfiguriert ist, um Informationen für jeden Zustand anzuzeigen mittels eines spezifischen Buchstabens, einer Kombination von Buchstaben, Texten und/oder eines grafischen Zeichens und/oder eines Symbols oder einer Kombination von Symbolen und/oder einer oder mehrerer bestimmter Farben oder anderer visueller/optischer Signale und Kombination von diesen.

9. Vorrichtung gemäß irgendeinem vorigen Anspruch, **dadurch gekennzeichnet, dass** die elektronische Zustandsanzeige (26) aufweist eine Bediener-Eingabeschnittstelle (36).

10. Vorrichtung gemäß Anspruch 9, **dadurch gekennzeichnet, dass** das Setzelement (40) die Eingabeschnittstelle (36) aufweist.

11. Vorrichtung gemäß irgendeinem vorigen Anspruch, **dadurch gekennzeichnet, dass** die elektronische Zustandsanzeige (26) konfiguriert ist, um Informationen bezüglich des spezifischen Zustands in einer Weise anzuzeigen, die nicht reversibel und/oder nicht modifizierbar ist ohne Autorisierung.

12. Vorrichtung gemäß irgendeinem vorigen Anspruch, **dadurch gekennzeichnet, dass** der Sensor (30) ausgewählt ist aus entweder einem Kontaktsensor oder einem Annäherungssensor.

13. Vorrichtung gemäß irgendeinem vorigen Anspruch, **dadurch gekennzeichnet, dass** das Setzelement (40) aufweist einen externen Stecker oder eine externe Steckdose (42), imstande das Senden und Empfangen eines elektrischen Leistungssignals oder von Daten oder einer Kombination von elektrischem Leistungs- und Daten-Signal zu unterstützen.

14. Vorrichtung gemäß Anspruch 13, **dadurch gekennzeichnet, dass** der Stecker oder die Steckdose (42) mit der elektronischen Zustandsanzeige (26) verbunden werden kann, wobei der Stecker oder die Steckdose (42) mit einer/einem zusammenpassenden, an einer Waschmaschine bereitgestellten Steckdose oder Stecker in Eingriff gebracht werden können, um mit der Verbindung der Verbinder (22) zusammenzufallen, wobei am Ende des Behandelns, wenn die Verbinder (22) getrennt werden, der Stecker oder die Steckdose (42) außer Eingriff gebracht werden können, oder Ende-des-Zyklus-Information empfangen werden kann, wobei ferner die Variation zwischen Im-Eingriff-Stehen und Außer-Eingriff-Stehen des Steckers oder der Steckdose (42), oder die Ende-des-Zyklus-Information, verwendet werden kann, um ein elektrisches und/oder Daten-Signal an die elektronische Zustandsanzeige (26) zu liefern, sodass es die Anzeige automatisch auf Behandlung bereits durchgeführt setzt.

15. Vorrichtung gemäß irgendeinem vorigen Anspruch, **dadurch gekennzeichnet, dass** die Verbinder (22) Schnellverbinder sind.

16. Vorrichtung gemäß irgendeinem vorigen Anspruch, **dadurch gekennzeichnet, dass**, wenn die Verbinder (22) nicht mit Hydraulikausgängen einer Waschmaschine für endoskopische Instrumente und/oder mit Verbindern (24) von medizinischen endoskopischen Instrumenten (12) verbunden sind, die Verbinder (22) geschlossen sind, verhindernd den Durchgang von Fluiden oder anderem Inneren des Aufnahmekörpers (14).

17. Vorrichtung gemäß irgendeinem vorigen Anspruch, **dadurch gekennzeichnet, dass** die Vorrichtung aufweist Umfangsdichtungen (19), um im geschlossenen Zustand die hermetische Abdichtung des Aufnahmekörpers (14) und des Schließkörpers (16) zu garantieren.

18. Verfahren zum Behandeln und Transportieren von medizinischen endoskopischen Instrumenten in einer geschützten Weise, aufweisend:
- Anordnen eines medizinischen endoskopischen Instruments (12) in einem Gehäuse (18, 65) eines Aufnahmekörpers (14, 62) und Schließen des Aufnahmekörpers (14, 62) mittels eines Schließkörpers (16, 55), der bezüglich des Aufnahmekörpers (14, 62) bewegbar ist, um einen hermetisch geschlossenen Zustand und einen offenen Zustand zu definieren,
**dadurch gekennzeichnet, dass** das Verfahren aufweist:
- Zuführen eines oder mehrerer Behandlungsfluide zu dem Gehäuse (18, 65) mittels eines oder mehrerer Hydraulikverbinder (22), die geeignet sind, mit unter Druck stehenden Flüssigkeiten zu arbeiten, und die verbindbar sind auf einer Seite mit hydraulischen Ausgängen einer Waschmaschine für endoskopische Instrumente und auf der anderen Seite: (i) mit Verbindern (24) von medizinischen endoskopischen Instrumenten, die in dem Gehäuse angeordnet sind, um Innenflächen der endoskopischen medizinischen Instrumente wiederaufzubereiten, und (ii) mit dem Inneren des Gehäuses, um die Außenflächen der medizinischen endoskopischen Instrumente wiederaufzubereiten,
- automatisches Erfassen einer Variation zwischen dem offenen Zustand und dem hermetisch geschlossenen Zustand und Senden eines korrespondierenden Signals mittels wenigstens eines Sensors (30), wobei der Sensor (30) ein Positionssensor ist und aufweist wenigstens zwei Sensorelemente (38), von denen ein Sensorelement (38) an dem Aufnahmekörper (14) angeordnet ist und ein Sensorelement (38) an dem Schließkörper (16) angeordnet ist, wobei die wenigstens zwei Sensorelemente (38) in einer abgestimmten und ausgerichteten Position angeordnet sind, sodass sie im geschlossenen Zustand des Schließkörpers (16) benachbart sind, wohingegen sie im offenen Zustand des Schließkörpers (16) im Abstand voneinander sind, wobei die gegenseitige Position der beiden Sensorelemente (38) variiert in Korrelation zu dem Übergang von dem geschlossenen Zustand zu dem offenen Zustand und umgekehrt, wobei diese Variation direkt mit dem Öffnen der Vorrichtung assoziiert ist, bereitstellend das Senden des korrespondierenden Signals,
- Empfangen des Signals von dem Sensor (30) und Anzeigen zur Außenseite hin von Informationen bezüglich des momentanen Zustands, der speziell mit dem empfangen Signal assoziiert ist, mittels einer elektronische Zustandsanzeige (26), wobei die elektronische Zustandsanzeige (26) mit einer Verarbeitungseinheit (32) bereitgestellt ist, die wenigstens ein Verarbeiten des Signals, das von dem Sensor (30) empfangen wurde, durchführt und eine Anzeige von Informationen bezüglich eines korrespondierenden Zustands befiehlt,
- Setzen eines Zustands komplettierten Behandelns des medizinischen endoskopischen Instruments (12) mittels eines Setzelements (40), sendend ein korrespondierendes Signal an die elektronische Zustandsanzeige (26).

## Revendications

1. Dispositif permettant de traiter et de transporter des instruments endoscopiques médicaux de manière protégée, comprenant un corps de confinement (14, 62), muni d'un logement (18, 65) destiné à la mise en place d'un instrument endoscopique médical (12), et un corps de fermeture (16, 55), dans lequel le corps de confinement (14, 62) et le corps de fermeture (16, 55) sont configurés mobiles l'un par rapport à l'autre, afin de définir une condition fermée de manière hermétique et une condition ouverte, **caractérisé en ce que** il comporte :
- un ou plusieurs connecteurs hydrauliques (22) adaptés pour travailler avec des liquides sous pression et pouvant être connectés d'un côté à des sorties hydrauliques d'une machine à laver pour instruments endoscopiques et de l'autre côté : (i) à des connecteurs (24) d'instruments médicaux endoscopiques placés dans ledit boîtier afin de retraiter les surfaces internes desdits instruments médicaux endoscopiques ; et (ii) à l'intérieur dudit boîtier afin de retraiter les surfaces externes desdits instruments endoscopiques médicaux ;
- au moins un capteur de position (30) configuré pour détecter automatiquement une variation entre la condition ouverte et la condition fermée de manière hermétique et pour transmettre un signal correspondant, ledit capteur de position (30) comprenant au moins deux éléments de capteur (38), dont un élément de capteur (38) disposé sur le corps de confinement (14) et un élément de capteur (38) disposé sur le corps de fermeture (16), lesdits au moins deux éléments de capteur (38) étant disposés dans une position coordonnée et alignée de sorte qu'ils se trouvent à proximité dans la condition fermée du corps de fermeture (16), tandis que dans la condition ouverte du corps de fermeture (16), ils sont éloignés l'un de l'autre, la position réciproque des deux éléments de capteur (38) variant en corrélation avec le passage de la condition fermée à la condition ouverte, et inversement, ladite variation étant directement associée à l'ouverture dudit dispositif, en fournissant l'émission dudit signal correspondant ;
- un affichage d'état électronique (26) configuré pour recevoir ledit signal à partir du capteur (30) et pour afficher à l'extérieur des informations concernant l'état actuel associé spécifiquement au signal reçu, ledit affichage d'état électronique (26) étant pourvu d'une unité de traitement (32) configurée au moins pour effectuer un traitement du signal reçu du capteur (30) et pour commander un affichage d'informations concernant un état correspondant ;
un élément de réglage (40) configuré pour définir un état de traitement terminé de l'instrument endoscopique médical (12) en transmettant un signal correspondant à l'affichage d'état électronique (26).

2. Dispositif selon la revendication 1, **caractérisé en ce que** ledit affichage d'état électronique (26) est configuré pour afficher au moins un état stérile de l'instrument endoscopique médical (12) associé à la condition fermée de manière hermétique, et un état non stérile de l'instrument endoscopique médical (12) associé à la condition ouverte et ou en cas de passage de la condition fermée de manière hermétique à la condition ouverte.

3. Dispositif selon la revendication 1 ou 2, **caractérisé en ce que** ledit affichage d'état électronique (26) est fourni sur le corps de fermeture (16) ou sur le corps de confinement (14).

4. Dispositif selon l'une quelconque des revendications précédentes, **caractérisé en ce qu'**il comporte une source d'énergie électrique (28) embarquée, configurée pour alimenter de manière autonome de l'énergie électrique au moins jusqu'à l'affichage d'état électronique (26).

5. Dispositif selon l'une quelconque des revendications précédentes, **caractérisé en ce que** l'affichage d'état électronique (26) comprend une ou plusieurs sources de lumière activées sélectivement.

6. Dispositif selon l'une quelconque des revendications précédentes, **caractérisé en ce que** l'affichage d'état électronique (26) comprend un écran d'affichage (34).

7. Dispositif selon l'une quelconque des revendications précédentes, **caractérisé en ce que** ledit écran d'affichage (34) est sensible au toucher.

8. Dispositif selon l'une quelconque des revendications précédentes, **caractérisé en ce que** l'affichage d'état électronique (26) est configuré pour afficher des informations pour chaque état au moyen d'une lettre spécifique, d'une combinaison de lettres, d'écritures et ou d'un signe graphique et/ou d'un symbole ou d'une combinaison de symboles et/ou d'une ou plusieurs couleurs dédiées ou d'autres signaux visuels/optiques, et de combinaisons de ceux-ci.

9. Dispositif selon l'une quelconque des revendications précédentes, **caractérisé en ce que** l'affichage d'état électronique (26) comprend une interface d'entrée d'utilisateur (36).

10. Dispositif selon la revendication 9, **caractérisé en ce que** ledit élément de réglage (40) comprend ladite interface d'entrée (36).

11. Dispositif selon l'une quelconque des revendications précédentes, **caractérisé en ce que** l'affichage d'état électronique (26) est configuré pour afficher des informations concernant l'état spécifique de manière irréversible et/ou non modifiable sans autorisation.

12. Dispositif selon l'une quelconque des revendications précédentes, **caractérisé en ce que** le capteur (30) est choisi parmi un capteur de contact ou un capteur de proximité.

13. Dispositif selon l'une quelconque des revendications précédentes, **caractérisé en ce que** ledit élément de réglage (40) comprend un connecteur ou une douille externe (42), capable de supporter la transmission et la réception d'un signal de puissance électrique, de données ou d'une combinaison de puissance électrique et d'un signal de données.

14. Dispositif selon la revendication 13, **caractérisé en ce que** ledit connecteur ou ladite douille (42) peut être connecté(e) à l'affichage d'état électronique (26) pour fournir le signal, dans lequel le connecteur ou la douille (42) peut être mis(e) en prise avec une douille ou un connecteur d'appariement prévu(e) sur une machine à laver, pour coïncider avec la connexion des connecteurs (22) et, à la fin du traitement, lorsque les connecteurs (22) sont déconnectés, le connecteur ou la douille (42) peut être libéré(e), ou des informations de fin de cycle peuvent être reçues, dans lequel en outre il est possible d'utiliser la variation entre la mise en prise et la libération du connecteur ou de la douille (42), ou des informations de fin de cycle, pour fournir un signal électrique et/ou de données à l'affichage d'état électronique (26) de sorte qu'il règle automatiquement l'affichage du processus déjà effectué.

15. Dispositif selon l'une quelconque des revendications précédentes, **caractérisé en ce que** lesdits connecteurs (22) sont des connecteurs de type rapide.

16. Dispositif selon l'une quelconque des revendications précédentes, **caractérisé en ce que** lorsque lesdits connecteurs (22) ne sont pas connectés à des sorties hydrauliques d'une machine à laver pour instruments endoscopiques et ou à des connecteurs (24) de l'instrument médical endoscopique (12), lesdits connecteurs (22) sont fermés, en empêchant le passage de fluides ou autres à l'intérieur du corps de confinement (14).

17. Dispositif selon l'une quelconque des revendications précédentes, **caractérisé en ce que** ledit dispositif comprend des garnitures de périmètre (19) pour garantir l'étanchéité hermétique du corps de confinement (14) et du corps de fermeture (16) dans la condition fermée.

18. Méthode pour traiter et transporter des instruments endoscopiques médicaux de manière protégée, comprenant les étapes consistant à :
mettre en place un instrument endoscopique médical (12) dans un logement (18, 65) d'un corps de confinement (14, 62) et fermer le corps de confinement (14, 62) au moyen d'un corps de fermeture (16, 55) mobile par rapport au corps de confinement (14, 62) afin de définir une condition fermée de manière hermétique et une condition ouverte,
**caractérisé en ce que** le procédé comprend les étapes consistant à :
- alimenter le boîtier (18, 65) en un ou plusieurs fluides de traitement au moyen d'un ou plusieurs connecteurs hydrauliques (22) adaptés pour travailler avec des liquides sous pression et pouvant être raccordés d'un côté aux sorties hydrauliques d'une machine à laver pour instruments endoscopiques et de l'autre côté : (i) à des connecteurs (24) d'instruments endoscopiques médicaux mis en place dans ledit logement afin de retraiter les surfaces internes desdits instruments endoscopiques médicaux ; et (ii) à l'intérieur dudit logement afin de retraiter les surfaces externes desdits instruments endoscopiques médicaux ;
- détecter automatiquement une variation entre la condition ouverte et la condition fermée de manière hermétique, et transmettre un signal correspondant au moyen d'au moins un capteur (30), ledit capteur (30) étant un capteur de position et comprenant au moins deux éléments de capteur (38), dont un élément de capteur (38) disposé sur le corps de confinement (14) et un élément de capteur (38) disposé sur le corps de fermeture (16), lesdits au moins deux éléments de capteur (38) étant disposés dans une position coordonnée et alignée de sorte qu'ils sont à proximité dans la condition fermée du corps de fermeture (16), tandis qu'ils sont éloignés dans la condition ouverte du corps de fermeture (16), la position réciproque des deux éléments de capteur (38) variant en corrélation avec le passage de la condition fermée à la condition ouverte, et inversement, ladite variation étant directement associée à l'ouverture dudit dispositif, en fournissant l'émission dudit signal correspondant;
recevoir ledit signal à partir du capteur (30) et afficher à l'extérieur des informations concernant l'état actuel, associé spécifiquement au signal reçu au moyen d'un affichage d'état électronique (26), ledit affichage d'état électronique (26) étant muni d'une unité de traitement (32) qui effectue au moins un traitement du signal reçu du capteur (30) et commande un affichage d'informations concernant un état correspondant ;
établir un état de traitement terminé de l'instrument endoscopique médical (12) au moyen d'un élément de réglage (40), et transmettre un signal correspondant à l'affichage d'état électronique (26).
